# EUROPEAN PATENT SPECIFICATION

(11) **EP 0 455 471 B1**
(45) Date of publication and mention of the grant of the patent: **08.11.1995**
(21) Application number: 91303918.6
(22) Date of filing: 30.04.1991
(51) Int. Cl.: G01N 33/52, C07D 417/12, C07D 409/12, C12Q 1/28

(54) **Improved chemiluminescent reagents and methods**
Verbesserte chemilumineszierende Reagenzien und Verfahren
Réactifs chimioluminescents améliorés et méthodes

(30) Priority: 30.04.1990 US 516330
(43) Date of publication of application: 06.11.1991
(73) Proprietor: Hitachi Chemical Co., Ltd., Shinjuku-ku, Tokyo 160 (JP)
(72) Inventor: Torkelson, Steven, San Mateo, CA (US); Kameda, Naomi, Woodside, CA (US)
(74) Representative: Goldin, Douglas Michael

(56) References cited:
- EP-A- 0 054 358
- EP-A- 0 087 959
- EP-A- 0 384 271

## Description

### Technical Field

The invention relates to the assays using chemiluminescence as an indicator with increase in light emission due to the presence of enhancer. More specifically, it relates to such luminescent assays which employ 2,3-dihydro-1,4-phthalazinediones rendered chemiluminescent, for example, through oxidation with an oxidant in the presence of catalyst, such as a peroxidase. The luminescence is enhanced by the presence of new phenolic enhancers. The stability of these reagents can also be improved by the addition of the enhancer in blocked form, if desired, so that it is released only in the context of the chemiluminescent reaction.

### Background Art

A widely employed detection system for a variety of assays, especially those involving specific binding of analyte, is that wherein a labeling moiety or binding partner is associated with a peroxidase, which in the presence of a 2,3-dihydro-1,4-phthalazinedione (DPD) substrate, such as luminol, and an oxidant results in chemiluminescence. While this is the most common type at present, other assays employing chemiluminescent indicators are also known, such as those which depend on the formation of an oxidant by analyte or which assess levels of peroxidase per se or luminol released into the reaction medium (see, e.g., U.S. patent 4,598,044). There is an extensive literature concerning the use of luminol-type chemiluminescent compounds in this manner, and although theoretically practical per se, the "luminol-based" detection system suffers from a low sensitivity unless a means to improve the light intensity is found. Such improvements have been suggested.

For example, U.S. patent 4,743,541 discloses a luminol preparation which is prepared from commercially available luminol by crystallization. Apparently this process eliminates luminescence inhibitors to the extent that a tenfold improvement in the intensity of luminescence can be obtained. The aforementioned patent specifically suggests the application of this purified form of luminol as a substrate in a now commercially available test to monitor the presence of antibody specific for various allergens in the serum of patients. In these tests, the suspected allergens are immobilized on a solid support and contacted with a sample of the serum to permit binding between the allergens and any antibodies present. The support is then contacted with a solution containing anti-IgE antibodies which have been labeled with peroxidase. The support is then contacted with a solution containing the purified luminol and peroxide, which behaves as a substrate mixture and causes the emission of light. This application of the substrate mixture is, of course, merely exemplary, and any assay which uses a peroxidase label can employ this chemiluminescent substrate mixture. Indeed, the chemiluminescence of various forms of luminol-related compounds can be used as an indicator in any assay where the presence or amount of analyte results in the excitation of the DPD with its subsequent return to a nonexcited state with the emission of light.

An alternate way to achieve the enhancement of chemiluminescence due to excitation of luminol and its related compounds is described and claimed in U.S. patents 4,598,044 and 4,729,950, which describes various aminoaromatic and phenolic compounds which enhance light production in this type of assay. These phenolic compounds and phenylamines are ring substituted in ways that are specifically defined. Additional embodiments of these enhancer compounds are described in European applications 087,959; 219,352; and 296,752. In the 087,959 application, the enhancers are various 6-hydroxybenzothiazoles; in EPO application 219,352, the enhancers are various phenylamines, and polycyclic aromatic amines substituted with electron donating substituents; and in EPO application 296,752, the enhancers are para-imidazol-1-yl phenols. US Patent 4,794,073 discloses a special application of enhancers in a chemiluminescent reaction wherein the enhancer is used as a label to detect the formation of a hybridized nucleic acid sequence.

An additional US Patent 4,835,101 describes an approach to dealing with the perceived problem of reagent stability when the substrate system is provided as a cocktail--i.e., wherein it contains the DPD, an oxidant, and enhancer. In this approach, the stability is maintained by lowering the pH of the cocktail to 3-6, rather than at about pH 8.5, which is optimum for light emission. The stability of the cocktail is said to be maintained even in the presence of the oxidant such as hydrogen peroxide, perborate ion, or urea-peroxide.

EP-A2-0384271 which was published on 29 August 1990 and has the priority of 14 February 1989 and which is citable under Article 54(3) EPC discloses a method for enhancing chemiluminescence which comprises using a compound of one of the following formulae: 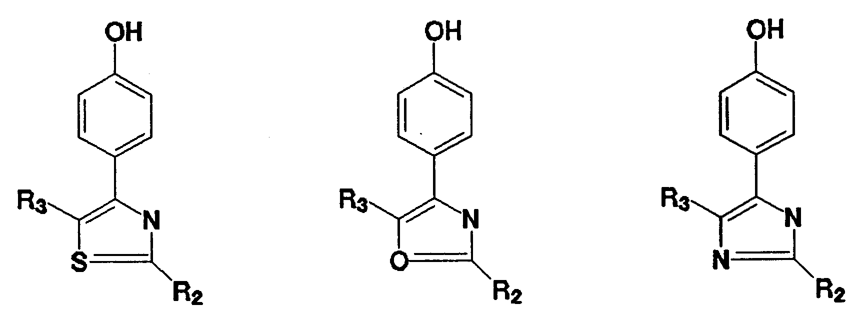
wherein R₂ and R₃ are each a hydrogen atom, halogen atom, a hydrocarbon residue which may be substituted or a heterocyclic group which may be substituted. EP-A1-0296752 discloses a method of enhancing the light output from a chemiluminescent reaction of a dihydrophthalazinedione (DPD), a peroxidase enzyme catalyst and an oxidant, in the presence of a phenolic compound as enhancer which enhancer comprises phenol substituted at its ortho and/or para position by an imidazolyl or benzimidazolyl group.

The present invention approaches maintenance of stability by using a blocked form of the enhancer, which is released upon conduct of the reaction, and provides additional enhancers which can be used in either blocked or unblocked forms.

### Disclosure of the Invention

The invention provides new enhancers and stabilized reagents for assays involving a detection system which employs the catalyzed oxidation of luminol-related compounds in the presence of an oxidant. New enhancer compounds are provided. The blocked enhancers contain phenolic compounds--such as those described in the patents and patent applications referenced in the Background section above, as well as the additional new enhancers described herein, in the form of an ester of a quaternized pyridine carboxylic acid--i.e., a picolinic or isonicotinic acid moiety wherein the positive charge on the ring nitrogen is stabilized by derivatization.

One aspect of the invention is directed to the use of improved enhancers, either in free or blocked form. These improved enhancing compounds are para-substituted phenols wherein the para-substituent is of the formula 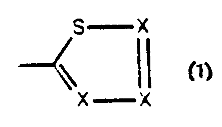
wherein each X is independently CR or N, wherein R is H or a moiety selected from the group consisting of R', OR', SR', NHR', NR'₂ and halo, wherein each R' is independently alkyl(1-6C); or wherein adjacent X=X may be substituted with -CR=CR-CR=CR-, wherein each R is H or a moiety as defined above.

Another aspect of the invention is directed to an improved method for conducting a luminescent or luminometric assay, wherein the improvement comprises conducting said reaction in the presence of the above-described enhancers, either in blocked or unblocked form.

In another aspect, the invention is directed to an improved method of conducting a luminescent or luminometric assay of any protocol, wherein the assay comprises conducting a reaction producing chemiluminescence by the interaction of an oxidizing catalyst, an oxidant, and a chemiluminescent DPD, and measuring or detecting the chemiluminescence produced, wherein the improvement is the addition of a blocked enhancer.

The blocked chemiluminescence enhancer is an aromatic ester of a carboxylic acid of the formula: 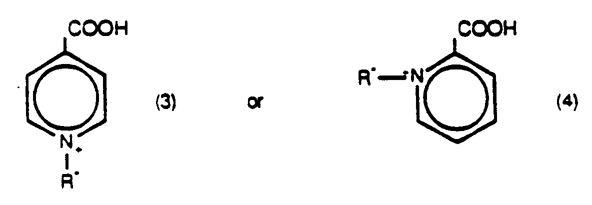
wherein R˝ is optionally substituted alkyl (1-6C). The composition may also include oxidant or a catalyst for oxidation, such as peroxidase. In addition, dye and/or buffer components may be present.

### Modes of Carrying Out the Invention

The compounds newly described as enhancers, and the innovation of use of a blocked enhancer are useful in any luminescent or luminometric assay involving the chemiluminescent indicator DPD. Various formats of this assay have been described in the art as set forth above. For example, the generation of an oxidant by the reaction of an analyte to be measured--most commonly, the generation of peroxide using substrates for oxidase enzymes--is used as an index for the appropriate substrate. In other instances, the oxidant or DPD or oxidizing catalyst may be released into the reaction medium in a manner that is dependent on analyte. In the most common application, however, the reaction is used to quantitate the amount of specific binding partner which is bound or unbound in a specific binding assay wherein the specific binding partner is labeled with the oxidizing catalyst, usually peroxidase.

The peroxidase label is very commonly used in a variety of such immunoassay techniques. A method to detect allergens for which the peroxidase label is particularly useful is described in a series of U.S. patents, 3,941,876 and 4,031,197, and an improved configuration for this assay is described in U.S. patents 4,558,013; 4,567,149; and 4,459,360.

A catalyst such as peroxidase may be used as a label by conjugation to a specific binding partner as described above, or may be used as a catalyst wherein the labeling compound is a DPD or an oxidant. Alternatively, the amounts or concentrations of the various components of the chemiluminescence reaction can be generated in response to analyte, as, for example, in the assay for glucose through generation of peroxide through the catalysis of glucose oxidase.

In general, therefore, the enhancers of the invention are applicable to any chemiluminescence assay which involves an oxidizing catalyst, an oxidant and a chemiluminescent DPD, wherein the chemiluminescence is a function of the analyte to be measured.

In general, such assays are conducted at pH in the range of 7.5-9, preferably about 8.5, to maximize chemiluminescence. This pH is generally achieved at the time all reagents are mixed.

The chemiluminescent DPD component is added at a concentration dependent on which component is being assayed; when catalyst or oxidant is the index of analyte, excess DPD is used. In general, the DPD concentration is 0.5 µM-200 mM, preferably 0.5-100 mM. Luminol is a preferred form of DPD.

If peroxidase is used as a catalyst, the oxidant is generally a peroxide or a perborate, and may advantageously be hydrogen peroxide-urea complex. The oxidant concentration is generally about 100 µM, which produces a high initial intensity that decreases rapidly with time; lower concentrations provide lower initial intensities but a less rapid decrease of the signal. The oxidant concentration is generally in the range of 0.5 µM-300 mM, preferably 10-200 mM.

The concentration of catalyst may be proportional to analyte, if used as label or an index of analyte. Where one of the other components is the index, and catalyst is used as a reagent, the concentration will depend on the nature of the catalyst but will be sufficient to be nonlimiting. Catalyst concentrations of 0.01 µg/l to 5 g/l, preferably not more than 50 mg/l.

The enhancers of the invention are provided in the range of 1 µM to 4 M, preferably 1 mM to 0.1 M.

### Preferred Embodiments of the New Enhancers of the Invention

The enhancers of the invention are para substituted phenols having substituents which are sulfur-containing aromatic 5-membered rings.

Preferred forms of the substituent of Formula 1 are those of Formulas 1a and 1c : 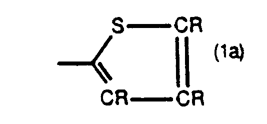 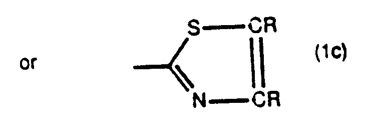
wherein each R is independently H or a moiety selected from the group consisting of -R', -OR', -SR', - NHR', -NR₂' and halo, wherein each R' is independently alkyl(1-6C), or wherein adjacent X=X may be substituted with -CR=CR-CR=CR-, wherein each R is H or a moiety as defined above.

As used herein, "alkyl" refers to a straight, branched chain or cyclic saturated hydrocarbyl moiety such as methyl, ethyl, isopropyl, isobutyl, N-pentyl, or cyclohexyl.

One group of preferred forms of the substituents of Formulas 1 and 2 are those of Formulas 1a', 1c', 2a' or 2d': 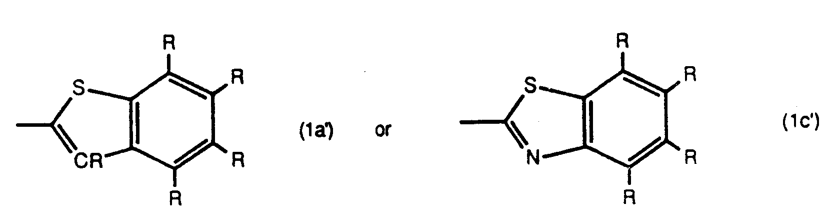
or those in general wherein each R is independently H or alkyl (1-6C). Thus, suitable compounds of the invention include, but are not limited to:
4-(2'-thienyl)phenol, and
4-[2'-(3'-ethyl)thienyl]phenol,
4-[2'-(3'-cyclohexyl)thienyl]phenol,
4-[2'-(4'-methyl)thienyl]phenol,
4-[2'-(3'-methoxy)thienyl]phenol,
4-[2'-(4'-diethylamino)thienyl]phenol,
4-[2'-(4'-butoxy)thiazolyl] phenol,
4-[2'-(4'-methoxy)thiazolyl] phenol,
4-[2'-(4'-cyclohexyl)thiazolyl] phenol,
4-[2'-(4'-propyl)thiazolyl] phenol,
4-[5'-(4'-butoxy)isothiazolyl] phenol,
4-[5'-(4'-propyl)isothiazolyl] phenol,
4-(2'-benzothiazolyl)phenol,
and
4-[2'-(6'-methoxy)benzothiazolyl]phenol.

Both the foregoing and conventional enhancer compounds, such as, for example, p-phenylphenol, p-iodophenol, 2,4-dibromophenol, 2,4-dichlorophenol, p-hydroxybenzoic acid or p-hydroxycinnamic acid can be used in blocked form so that they are stabilized until released by the conditions of the chemiluminescent assay. A suitable blocked form utilizes these phenolic enhancers as esters of stabilized forms of picolinic or isonicotinic acid of formulas 3 and 4; i.e., compounds of the formula 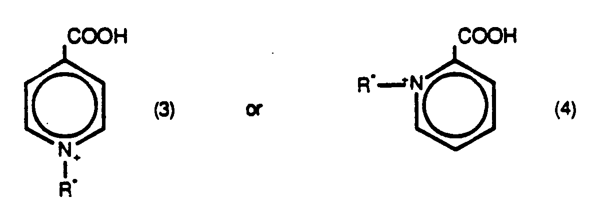
wherein R˝ is an optionally substituted alkyl substituent (1-6C), with a phenolic enhancer.

These acids are used as salts of bases with the ring nitrogen; the ring nitrogen is a cationic quaternary amine. Convenient salts include the fluorosulfonate, iodide and methyl sulfonate salts.

The esters are prepared using conventional esterification techniques to provide the blocked enhancers. Typical conditions include preparing the activated form of the picolinic or isonicotinic acid, such as by treatment with thionyl chloride. The activated form can then be directly reacted with the phenol to obtain the desired ester.

The following examples are intended to illustrate but not to limit the invention.

### Example 1

### Preparation of 4-(2′-Thienyl)phenol

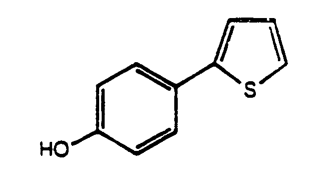

A. The preparation of this compound is according to the method of Gopinathan, S., et al. Ind J Chem (1974) 12:623.

2-Iodothiophene (20.0 mmol) in absolute ether was added dropwise to a stirred to a stirred suspension of magnesium turnings (25.0 mmol) in ether, in an atmosphere of dry nitrogen. Vigorous reaction took place and the reagent (in ether solution) was ready for use with 30 min.

To the cooled and stirred Grignard reagent was added (dropwise) 16.0 mmol of tributyl tin chloride in ether. The mixture was refluxed for one hour and ether was removed after adding benzene. The excess Grignard reagent was destroyed by adding water and the benzene layer was separated. This was dried and evaporated to yield a dark brown oil which was distilled under vacuum to yield 4.8 g (80%) of the tributylthienyl tin compound; bp 150°/1.0 mm.

The tin compound (10.0 mmol), 4-iodoanisole (8.7 mmol), PdCl₂(PPh₃)₂ (0.87 mmol) were dissolved in 18.0 ml of anhydrous toluene and refluxed gently under N₂ for 20 hour, according to the procedure of Kosugi, M., et al., Bull Chem Soc Japan (1986) 59:677; and Bailey, Tet Lett (1984) 4407. The reaction mixture turned black as palladium metal was formed. The mixture was cooled and filtered through a large pad of neutral alumina (eluant ether). The filtrate was washed several times with water and the ether phase was dried (MgSO₄). Concentration led to an oil which crystallized upon addition of methanol/water. The solid product was recrystallized from methanol/water to yield 1.0 g (61%), mp 103-106°.

A mixture of 1.0 g (5.3 mmol) of the above ether was cleaved with 10 g of pyridine hydrochloride according to the procedure of Baldwin, L.J., et al., J Heterocycl Chem, (1985) 22:1667, by heating the mixture at 200° for 12 hours. After the mixture cooled, 50 ml of water was added and the resultant precipitate was isolated by filtration. The solid was dissolved in 20% sodium hydroxide solution, filtered, and neutralized with acetic acid. The white solid title compound was filtered and recrystallized from methanol/water; mp 146-148°.

B. Alternate method: A preferred method to prepare the title compound is described in Baldwin et al. (supra) and is conducted as follows:
A solution of thiophene (5.76g, 68.0 mmol) was dissolved in 20 ml of anhydrous ether and was cooled to 0°C in an ice bath. The thiophene was lithiated at 0°C by adding dropwise 21.2 ml of 1.6M n-butyllithium solution in hexane. After the addition, the solution was stirred at 0°C for 30 min to assure complete lithiation. Cuprous iodide (6.48g, 34.0 mmol) was added and the mixture was allowed to warm to room temperature over the period of one hr. After one additional hr, 8.0g (34.0 mmol) of 4-iodoanisole and 60 ml of dry quinoline were added and the ether and hexane was distilled off. The reaction mixture was heated to 150°C for 3 hr. The mixture was then cooled and 80 ml of ether was added. The insoluble salts were filtered off and washed with ether. The ether was washed with 10% aqueous hydrochloric acid solution. After washing with water, the ether layer was dried and evaporated to yield a solid residue which was dissolved in benzene-hexane (60:50) and subjected to a chromatographic separation on a neutral alumina column. The product was dissolved in hot methanol and water was added. Cooling of the mixture yielded a voluminous mass of crystals yield 5.82g (90%); mp 104-106°.

The above ether (1.5g, 7.8 mmol) was mixed with 1.50g of lithium thioethoxide in 20 ml of dry dimethylformamide (DMF) and heated to 150°C for 1 1/2 hr. The mixture was poured onto water, washed with ether, the ether extracted with 10% aqueous sodium hydroxide solution and this was added to the previous water layer. Acidification of the alkaline aqueous layer with 10% aqueous hydrochloric acid yields the free phenol which is extracted with ethyl acetate. After drying and evaporation, the DMF was removed under high vacuum by azeotroping it with water. Chromatography on a silica gel column (ethyl acetate) gave a fairly pure title compound which is crystallized from methanol/water, yield 0.80g (58%); mp 146-148°C.

### Example 2

### Preparation of 4-[2'-(4'-Methyl)thiazolyl]phenol

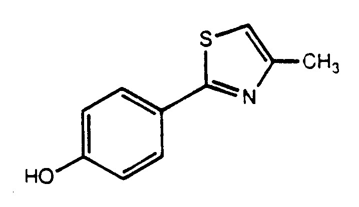

This compound was prepared as described by Suter, C.M., et al., J Am Chem Soc (1930) 52:1585. 4-Methoxybenzonitrile (1.0 g 7.5 mmol) was dissolved in 6.0 ml ethanol and 2.0 ml of 29% ammonium hydroxide was added. The solution was saturated with hydrogen sulfide and the flask was closed. It was heated at 100° for 1 hr and cooled. Upon evaporation, a yellowish residue remained. This was crystallized from hot water to yield the thioanisamide as yellow leaflets, yield 0.98 g (78%) mp 148-149°.

Thioanisamide (1.67 g, 10.0 mmol) and chloroacetone (1.02 g, 11.0 mmol) in 8.0 ml of absolute alcohol were heated at reflux for 1 hr. After cooling, the hydrochloride of the thiazole was precipitated from this solution by dilution with ether. The yield was 1.84 g (90%). The product was demethylated as is.

The hydrochloride salt from the above procedure (1.84 g) was dissolved in 30.0 ml of 48% hydrobromic acid and refluxed for 2 hr. After cooling, the mixture was diluted with water and basified to pH 8. After filtration and drying it was recrystallized from methanol and methanol/water to yield 1.55 g (90%) of the pure title compound; mp 220-221°.

## Claims

1. A luminescent or luminometric assay which comprises conducting a chemiluminescent reaction using an oxidation catalyst, an oxidant, and a chemiluminescent 2,3-dihydro-1,4-phthalazinedione, and measuring or detecting the chemiluminescence thereby produced, wherein the reaction is carried out in the presence of a para-substituted phenolic enhancer wherein the para-substituent is of the formula 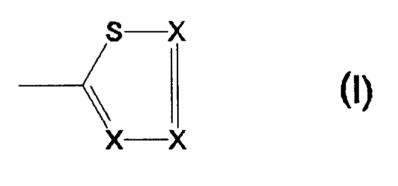 wherein each X is independently CR or N; wherein R is H or a moiety selected from the group consisting of R', OR', SR', NHR', NR'₂ and halo, wherein each R' is independently alkyl(1-6C); or wherein adjacent X=X may be substituted with -CR=CR-CR=CR-, wherein each R is H or a moiety as defined above.

2. The assay of claim 1 wherein the substituent of formula 1 has the formula: 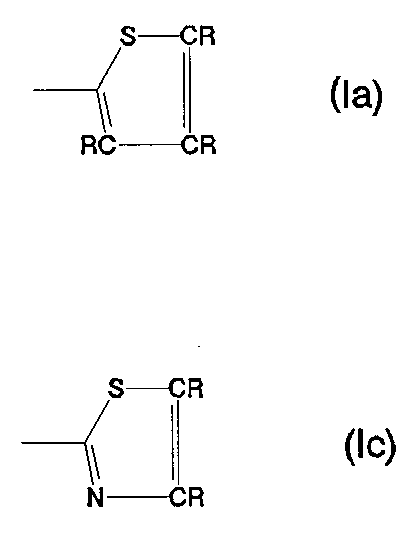 wherein R is as defined in claim 1.

3. The assay of claim 1 or 2 wherein each R is independently H or alkyl (1-6C).

4. The assay of claim 2 wherein the substituent of formula (1a) has the formula:

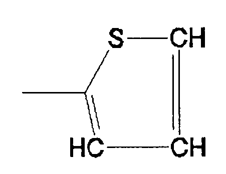

5. The assay of claim 2 wherein the substituent of formula (1c) has the formula:

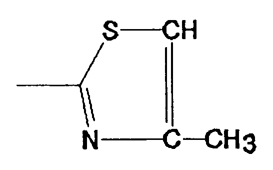

6. A composition useful to prepare a substrate reagent for a chemiluminescence-based reaction, which composition comprises a chemiluminescent 2,3-dihydro-1,4-phthalazinedione and a para-substituted phenolic enhancer wherein the para-substituent is of the formula (1) defined in any one of the preceding claims.

7. A blocked enhancer useful in enhancing the emission of light in a luminescent or a luminometric assay which is an ester formed by a compound of the formula: 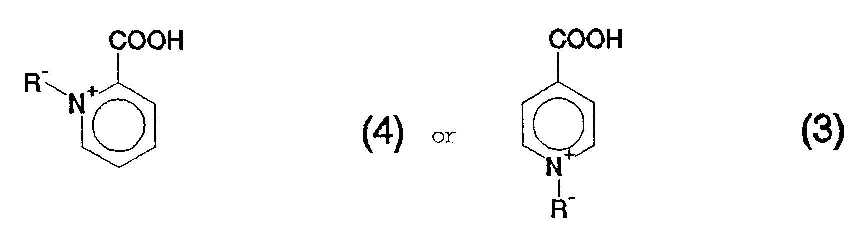 and an alcohol which is a para-substituted phenolic enhancer wherein the para-substituent is of formula (1) defined in any one of claims 1 to 5.

## Patentansprüche

1. Lumineszens- oder Luminometrie-Assay, dadurch **gekennzeichnet,** daß unter Verwendung eines Oxidationskatalysators, eines Oxidationsmittels und eines chemilumineszierenden 2,3-Dihydro-1,4-phthalazindions eine chemilumineszierende Reaktion durchgeführt wird, die dabei gebildete Chemilumineszens gemessen oder nachgewiesen wird, wobei die Reaktion in Anwesenheit eines para-substituierten phenolischen Enhancers durchgeführt wird, wobei der Para-Substituent die Formel 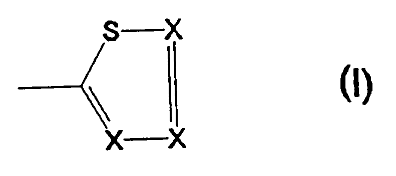 besitzt, worin jedes X unabhängig CR oder N bedeutet; worin R H oder eine Gruppierung, ausgewählt aus der Gruppe, bestehend aus R', OR', SR', NHR', NR'₂ und Halogen bedeutet, worin jedes R' unabhängig Alkyl-(1-6C) bedeutet; oder worin benachbarte X=X mit -CR=CR-CR=CR- substituiert sein können, worin jedes R H oder die oben definierte Gruppierung bedeutet.

2. Assay nach Anspruch 1, worin der Substituent der Formel 1 die Formel 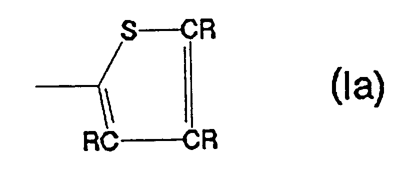 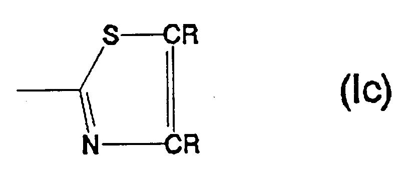 besitzt, worin R die in Anspruch 1 gegebene Definition besitzt.

3. Assay nach Anspruch 1 oder 2, worin jedes R unabhängig H oder Alkyl-(1-6C) bedeutet.

4. Assay nach Anspruch 2, worin der Substituent der Formel (1a) die Formel 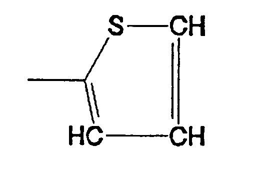 besitzt.

5. Assay nach Anspruch 2, worin der Substituent der Formel (1c) die Formel 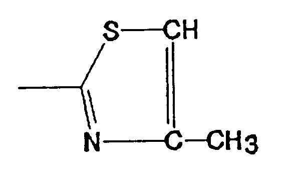 besitzt.

6. Zusammensetzung, die zur Herstellung eines Substratreagens für eine Reaktion auf der Grundlage von Chemilumineszens nützlich ist, wobei die Zusammensetzung ein chemilumineszierendes 2,3-Dihydro-1,4-phthalazindion und einen para-substituierten phenolischen Enhancer enthält, worin der Para-Substituent die Formel (1), wie in irgendeinem der vorhergehenden Ansprüche definiert, besitzt.

7. Inaktivierter Enhancer, der für die Verstärkung der Emulsion von Licht in einem Lumineszens- oder Luminometrie-Assay geeignet ist, der ein Ester ist, gebildet aus einer Verbindung der Formel 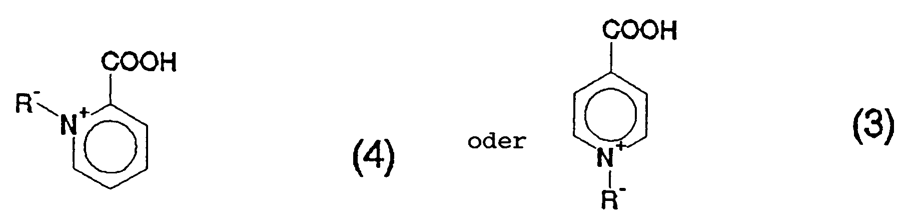 und einem Alkohol, der ein para-substituierter phenolischer Enhancer ist, worin der Para-Substituent die Formel (1), wie in irgendeinem der Ansprüche 1 bis 5 definiert, besitzt.

## Revendications

1. Essai luminométrique ou par luminescence, qui comporte le fait de réaliser une réaction chimiluminescente en utilisant un catalyseur d'oxydation, un oxydant et une 2,3-dihydro-1,4-phtalazinedione chimiluminescente et le fait de détecter ou de mesurer la chimiluminescence ainsi produite, cette réaction étant effectuée en présence d'un renforçateur qui est un composé phénolique para-substitué, dans lequel le substituant placé en para correspond à la formule : 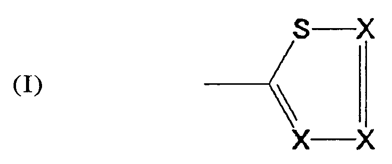 dans laquelle chaque symbole X représente indépendamment N ou CR où R représente un atome H ou un fragment choisi dans l'ensemble formé par les atomes d'halogène et les groupes symbolisés par R', OR', SR', NHR' et NR'₂ où R' représente, indépendamment dans chaque cas, un groupe alkyle en C₁₋₆, ou dans laquelle les symboles adjacents X=X peuvent ensemble porter un substituant de formule -CR=CR-CR=CR- où chaque R représente un atome H ou un fragment tel que défini ci-dessus.

2. Essai conforme à la revendication 1, dans lequel le substituant de formule (I) correspond à la formule 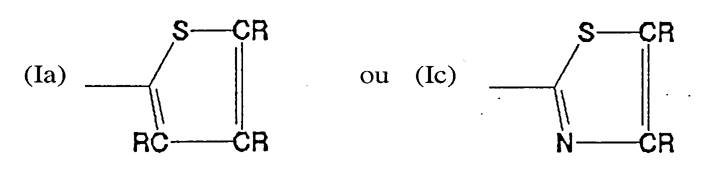 où R a la signification indiquée dans la revendication 1.

3. Essai conforme à la revendication 1 ou 2, dans lequel chaque R représente indépendamment un atome H ou un groupe alkyle en C₁₋₆.

4. Essai conforme à la revendication 2, dans lequel le substituant de formule (Ia) correspond à la formule

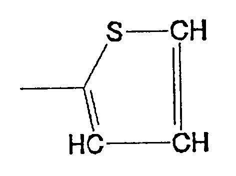

5. Essai conforme à la revendication 2, dans lequel le substituant de formule (Ic) correspond à la formule

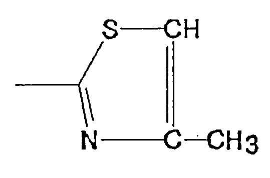

6. Composition utilisable pour préparer un réactif jouant le rôle de substrat dans une réaction chimiluminescente, laquelle composition comprend une 2,3-dihydro-1,4-phtalazinedione chimiluminescente et un renforçateur qui est un composé phénolique para-substitué, dans lequel le substituant placé en para correspond à une formule (I) définie dans l'une quelconque des revendications précédentes.

7. Renforçateur inhibé, utilisable pour renforcer l'émission de lumière au cours d'un essai luminométrique ou par luminescence, qui est un ester formé par un composé de formule 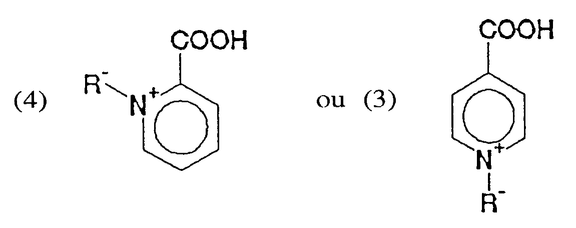 et un alcool qui est un composé phénolique para-substitué renforçateur dont le substituant placé en para correspond à une formule (I) définie dans l'une quelconque des revendications 1 à 5.
